# EUROPEAN PATENT SPECIFICATION

(11) **EP 4 272 678 B1**
(45) Date of publication and mention of the grant of the patent: **10.06.2026**
(21) Application number: 23171115.1
(22) Date of filing: 02.05.2023
(51) Int. Cl.: A61B 34/10

(54) **ABLATION ASSISTANCE USING NUMERIC MODELING**
ABLATIONSUNTERSTÜTZUNG UNTER VERWENDUNG VON NUMERISCHER MODELLIERUNG
ASSISTANCE D'ABLATION UTILISANT UNE MODÉLISATION NUMÉRIQUE

(30) Priority: 03.05.2022 US 202263364097 P; 11.04.2023 US 202318298731
(43) Date of publication of application: 08.11.2023
(73) Proprietor: Medtronic, Inc., Minneapolis, MN 55432-5604 (US)
(72) Inventor: BECKMAN, Mary A., Mounds View, 55112 (US); O'CONNELL, Heather L., Mounds View, 55112 (US); LASKE, Timothy G., Mounds View, 55112 (US); STEWART, Mark T., Mounds View, 55112 (US); HOWARD, Brian T., Mounds View, 55112 (US); MATTISON, Lars M., Mounds View, 55112 (US); SCHMIDT, Megan M., Mounds View, 55112 (US); AHLBERG, Sarah E., Mounds View, 55112 (US)
(74) Representative: Zimmermann & Partner Patentanwälte mbB

(56) References cited:
- US-A1- 2017 209 220
- US-A1- 2019 223 955
- US-A1- 2020 375 555
- US-A1- 2021 065 906
- US-A1- 2021 137 606
- US-A1- 2022 008 126

## Description

This application claims the benefit of U.S. Provisional Patent Application Ser. No. 63/364,097, entitled, "ABLATION ASSISTANCE USING NUMERIC MODELING," and filed on May 3, 2022.

### TECHNICAL FIELD

The present technology is related to ablation catheters. In particular, various examples of the present technology are related to assisting ablation using one or more ablation catheters and numerical or computational modeling.

### BACKGROUND

Tissue ablation is a medical procedure commonly used to treat conditions such as cardiac arrhythmias, which includes atrial fibrillation. For treating cardiac arrhythmias, ablation can be performed to modify tissue, such as to stop aberrant electrical propagation and/or disrupt aberrant electrical conduction through cardiac tissue. Ablation techniques include pulsed field ablation (PFA), cryoablation, laser ablation, thermal ablation, radioablation and radiofrequency (RF) ablation.

Cardiac arrhythmias are a group of conditions that cause an irregular heartbeat or conduction pattern. Ablation may be used to create a safe and effective lesion or set of lesions at the origin of the irregular heartbeat or in regions that aid in the termination of arrhythmias without causing damage to adjacent structures or surrounding tissue, ideally resulting in no need for a maintenance treatment regimen, such as medications or cardioversions. Document US 2021/065906
Al relates to calibration of simulated cardiograms. Document US 2020/375555 A1 relates to cardiac electrical mapping and ablation. Document US 2022/008126 A1 relates to optimized ablation for persistent atrial fibrillation. Document US 2019/223955 relates to denervation therapy. Document US 2021/137606 Al relates to assisting in positioning a thermal ablation device.

### SUMMARY

The present technology is directed to devices, systems, and methods for assisting ablation using one or more ablation catheters and numerical modeling. As used herein a "numerical model" or "numerical modeling" may include a computational model or computational modeling. Each ablation patient has a unique anatomy. Techniques that combine anatomical, computational and/or electrical characterization with patient history to identify where to ablate and one or more preferred energy modalities, and confirm successful ablation, are desirable as they may increase efficacy, safety, and efficiency of the ablation procedure. Techniques disclosed herein may be used to show clear indicators as to which tissue to ablate or which tissue will be ablated, accurate endpoints of when a physician should reliably stop ablating the tissue and increase the confidence level that a successful lesion (e.g., one that may mitigate or stop the cardiac arrythmia) was created.

In accordance with one or more aspects of this disclosure, a system for use in ablating target tissue of a patient includes: memory configured to store at least one of anatomical information of a patient or physiological information of the patient and a numerical model; and processing circuitry communicatively coupled to the memory, the processing circuitry being configured to: determine, based on the at least one of the anatomical information of the patient or the physiological information of the patient, ablation parameters using the numerical model, the ablation parameters comprising at least one of a suggested positioning of at least one energy delivery element of at least one catheter or an amount of energy to be delivered via the at least one energy delivery element to target tissue of the patient during ablation; and output, for display, a representation of at least one of the suggested positioning of the at least one energy delivery element during the ablation, a representation of the target tissue that would be ablated after delivery of ablation energy via the at least one energy delivery element, or a representation of predicted tissue that will be ablated after the delivery of the ablation energy via the at least one energy delivery element.

In another example, a method includes determining, by processing circuitry and based on at least one of anatomical information of a patient or physiological information of the patient, ablation parameters using a numerical model, the ablation parameters comprising at least one of a suggested positioning of at least energy delivery element of at least one catheter or an amount of energy to be delivered via the at least one energy delivery element to target tissue of a patient during ablation; and outputting, for display by the processing circuitry, a representation of at least one of the suggested positioning of the at least one energy delivery element during the ablation, a representation of the target tissue that would be ablated after delivery of ablation energy via the at least one energy delivery element, or a representation of predicted tissue that will be ablated after the delivery of the ablation energy via the at least one energy delivery element

In another example, a non-transitory computer-readable storage medium stores instructions that, when executed cause processing circuitry to determine, based on the at least one of anatomical information of the patient or physiological information of the patient, ablation parameters using the numerical model, the ablation parameters comprising at least one of a suggested positioning of at least one energy delivery element of at least one catheter or an amount of energy to be delivered via the at least one energy delivery element to target tissue of the patient during ablation; and output, for display, a representation of at least one of the suggested positioning of the at least one energy delivery element during the ablation, a representation of the target tissue that would be ablated after delivery of ablation energy via the at least one energy delivery element, or a representation of predicted tissue that will be ablated after the delivery of the ablation energy via the at least one energy delivery element.

Further disclosed herein is an example system for use in ablating target tissue. An example system for use in ablating target tissue includes memory configured to store anatomical and/or physiological information of a patient and processing circuitry communicatively coupled to the memory. The processing circuitry is configured to, based on the anatomical information and/or the physiological information, determine ablation parameters, the ablation parameters including a suggested positioning of at least energy delivery element of at least one catheter and/or an amount of energy to be delivered via the at least one energy delivery element to the target tissue during ablation. The processing circuitry is configured to output, for display, a representation of at least one of a suggested positioning of the at least one energy delivery element during the ablation, a representation of the target tissue, or a representation of the predicted tissue volume that will be ablated after delivery of ablation energy

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

FIG. 1 is a conceptual diagram illustrating an example system for delivering ablation.
FIG. 2 is a block diagram illustrating an example controller of an ablation system, in accordance with one or more aspects of this disclosure.
FIGS. 3A-3D are conceptual diagrams illustrating examples of information displayed by a user interface in accordance with one or more techniques of this disclosure.
FIG. 4 is a flowchart illustrating an example ablation assistance technique in accordance with one or more techniques of this disclosure.
FIG. 5 is a conceptual diagram illustrating an example machine learning model in accordance with one or more techniques of this disclosure.
FIG. 6 is a conceptual diagram illustrating an example training process for a machine learning model in accordance with one or more techniques of this disclosure.

### DETAILED DESCRIPTION

Based on qualitative and quantitative research, ablation clinicians, such as cardiac electrophysiologists, have expressed a desire of providing higher confidence in the efficacy of lesions that they create during a cardiac ablation procedure. Thus, it may be desirable to improve or maximize the ability of a clinician to make an effective lesion and confirm that they have made an effective lesion. In order to improve clinician confidence that they are ablating the appropriate target tissue, clinicians may want to know where to ablate, an effective energy level with which to ablate, and be able to confirm that the ablation did what the clinician intended the ablation to do (e.g., ablate the appropriate target tissue and deliver a lesion of appropriate size). By providing such information to a clinician, the techniques of this disclosure may reduce or minimize the occurrences of gaps when isolating the pulmonary veins (PVs) and/or creating non-PV lesions, as well as improve or maximize the likelihood of the clinician creating consistent and transmural lesions regardless of any variation in tissue thickness. By providing such information to a clinician, patient outcomes from ablation may be improved.

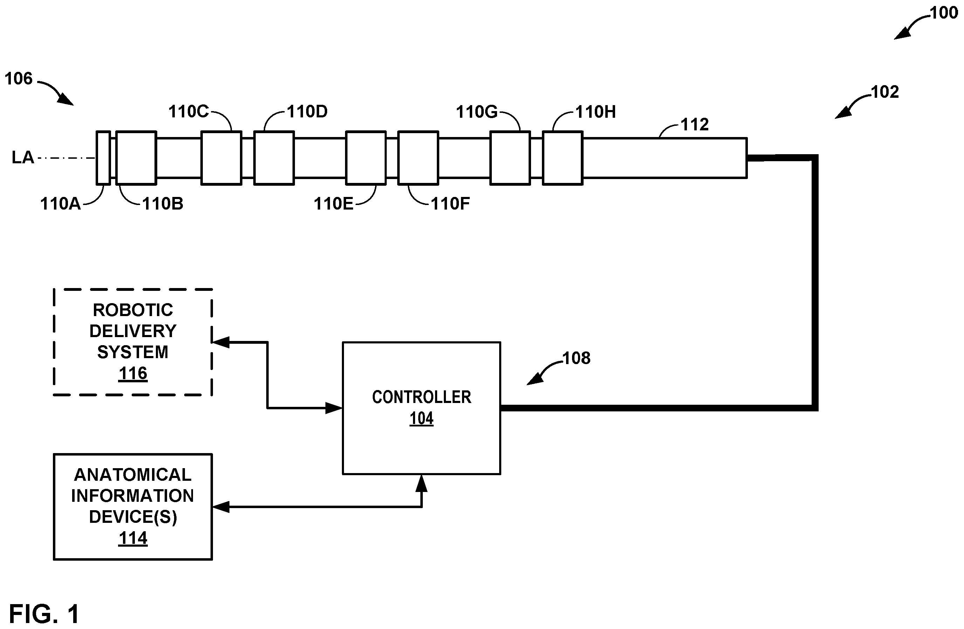

FIG. 1 is a conceptual diagram illustrating an example system for delivering ablation. System 100 includes a catheter 102, a controller 104, an anatomical information device 114, and optionally (shown in dashed lines), a robotic delivery system 112. In general, to deliver ablation, a practitioner (e.g., electrophysiologist, interventional cardiologist, etc.) may insert one or more of catheter 102 into a patient and cause controller 104 to deliver, via catheter 102, energy (e.g., pulsed field ablation energy, radiofrequency ablation energy, laser ablation, thermal ablation, radioablation, carbon ion beam ablation, or cryoablation energy) to target tissue of a patient. Ablation may cause lesions in target cardiac tissue which may mitigate or stop cardiac arrhythmias. As ablation causes lesions in the heart, it may be desirable for a system to safely and accurately provide information to a clinician regarding ablation parameters, such as a suggested location where to position one or more energy delivery elements of one or more ablation catheters, an amount of energy to be delivered during ablation, or which tissue would be ablated after delivery of ablation energy via the at least one energy delivery element. In some examples, rather than, or in addition to, providing information as to where to position one or more energy delivery elements, controller 104 may control robotic delivery system 116 to deliver catheter 102 into anatomy of a patient such that the one or more energy delivery element may be positioned at the suggested location. In some examples, controller 104 may include robotic delivery system 116.

Catheter 102 may include elongated structure 112 carrying a plurality of energy delivery elements 110A-110H (collectively "energy delivery elements 110"). An energy delivery element may include an electrode (e.g., in the case of a pulsed field ablation catheter), a cryogenic element (e.g., in the case of a cryoablation catheter), a radiofrequency element (e.g., in the case of a radiofrequency ablation catheter), or another energy delivery element. While the techniques of this disclosure are applicable to any ablation catheter, the example of FIG. 1 is directed to a pulsed field ablation catheter. Catheter 102 may generally include features that enable insertion of catheter 102 into a patient and navigation of catheter 102 to a target tissue site. Elongated structure 112 may include a distal portion 106 and a proximal portion 108. Energy delivery elements 110 may be generally positioned at distal portion 106, while proximal portion 108 may be connected to controller 104. Energy delivery elements 110 may be of any suitable geometry. Example geometries of electrodes include, but are not necessarily limited to, circular (e.g., ring) electrodes surrounding the body of catheter 102, conformable electrodes, cuff electrodes, segmented electrodes (e.g., electrodes disposed at different circumferential positions around catheter 102 instead of a continuous ring electrode), any combination thereof (e.g., ring electrodes and segmented electrodes). Energy delivery elements 110 may be axially distributed along longitudinal axis LA of elongated structure 112 or in several other configurations. In some examples, catheter 102 may include one or more energy delivery elements 110 and the geometry of the one or more energy delivery elements 110 may include a balloon, which may be inflated when performing ablation and deflated when navigating catheter 102 to the target tissue. The delivery elements 110 may also be in a circular form, in an array, along multiple splines, or in other configurations.

Elongated structure 112 may include conductors configured to carry electrical signals between energy delivery elements 110 and controller 104. In some examples, elongated structure 112 may include a separate conductor for each of energy delivery elements 110. For instance, in the example of FIG. 1 where energy delivery elements 110 includes eight electrodes, elongated structure 112 may include eight separate conductors. In this way, elongated structure may enable each electrode of energy delivery elements 110 to be driven with a different signal from controller 104. In other examples, multiple electrodes of energy delivery elements 110 may share a common conductor. For instance, energy delivery elements 110C and 110D may be connected to a same (e.g., a common) conductor. While such a common conductor arrangement may reduce energy delivery element flexibility (e.g., as electrodes connected to the common conductor may be driven with a same signal), such an arrangement may reduce manufacturing complexity and/or cost and may increase the structural flexibility of catheter 102.

As shown in FIG. 1, energy delivery elements 110 may include a tip electrode (e.g., electrode 110A), which may be a ring electrode with a "cap" covering at least a portion of a tip of elongated structure 112. In some examples, the tip electrode may be chamfered or otherwise rounded (e.g., to enable easier passage of catheter 102 through anatomy of the patient). Energy delivery elements 110 may include a ring electrode (e.g., electrode 110B) that is adjacent to the tip electrode. This ring electrode may be separated (axially along LA) from the tip electrode. Energy delivery elements 110 may include one or more pairs of ring electrodes. A pair of ring electrodes may include two adjacently closely spaced electrodes of energy delivery elements 110. For instance, in the example of FIG.1, energy delivery elements 110C and 110D may form a first pair of ring electrodes, energy delivery elements 110E and 110F may form a second pair of ring electrodes, and energy delivery elements 110G and 110H may form a third pair of ring electrodes. In general, the first pair of ring electrodes (i.e., energy delivery elements 110C and 110D) may be accompanied by one or more additional electrodes. The one or more additional electrodes may include any combination of pairs of ring electrodes and coil electrodes (e.g., electrodes that include conductors that spiral around elongated structure 112).

In the example of FIG. 1, energy delivery elements 110 are illustrated as has having a larger diameter than elongated structure 112. In some examples, one or more of energy delivery elements 110 may have a diameter that is approximately equal to or less than the diameter of elongated structure 112. For instance, energy delivery elements 110 may be recessed in elongated structure 112 such that the combination results in a relatively smooth outer surface.

Controller 104 may include an energy generator configured to provide electrical pulses to energy delivery elements 110 (or to control the delivery of radio frequency energy or cryogenic energy by energy delivery elements 110) to perform an ablation procedure to cardiac tissue or other tissues within the patient's body, such as renal tissue, airway tissue, and organs or tissue within the cardiac space or the pericardial space. For instance, the energy generator may be configured and programmed to deliver pulsed, high-voltage electric fields appropriate for achieving desired pulsed, high-voltage ablation (referred to as "pulsed field ablation" or "pulsed electric field ablation") and/or pulsed radiofrequency ablation. In some examples, the energy generator may be configured and programmed for achieving desired cryogenic ablation.

Anatomical information device(s) 114 may include a computed tomography (CT) device, a magnetic resonance imaging (MRI) device, an ultrasound (U/S) device, a pacing device, an electrophysiology (EP) mapping device, and/or a non-invasive mapping device. Anatomical information device(s) 114 may be used by system 100 to inform controller 104 of the anatomy of a patient, physical or electrical characteristics of the anatomy, and/or a location of catheter 102 during delivery of catheter 102 into the anatomy of a patient. In some examples, controller 104 may include one or more of anatomical information device(s) 114.

In accordance with the techniques of this disclosure, a system 100 for use in ablating target tissue (e.g., a target tissue area or volume) of a patient includes memory configured to store at least one of anatomical information of a patient or physiological information (which may include cardiac electrophysiological information) of the patient, and a numerical model and processing circuitry (e.g., of controller 104) communicatively coupled to the memory. The processing circuitry may be configured to: determine ablation parameters using the numerical model and based on the one of the anatomical information of the patient or the physiological information of the patient, the ablation parameters comprising at least one of a suggested positioning of at least one energy delivery element (e.g., of energy delivery elements 110) of at least one catheter (e.g., catheter 102) or an amount of energy to be delivered via the at least one energy delivery element to the target tissue during ablation; and output for display a representation of at least one of the suggested positioning of the at least one energy delivery element during the ablation, a representation of the target tissue that will be ablated after delivery of ablation energy via the at least one energy delivery element, or a representation of predicted tissue that will be ablated after the delivery of the ablation energy via the at least one energy delivery element.

The techniques of this disclosure may provide clinicians higher confidence in the efficacy of lesions that they create during a cardiac ablation procedure and improve or maximize the ability of a clinician to make an effective lesion and confirm that they have made an effective lesion. The techniques of this disclosure may reduce or minimize the occurrences of gaps when isolating the pulmonary veins (PVs) and/or creating non-PV lesions, as well as improve or maximize the likelihood of the clinician creating consistent and transmural lesions regardless of any variation in tissue thickness. By providing such information to a clinician, patient outcomes from ablation may be improved.

Although not shown, system 100 may include one or more sensors to monitor the operating parameters through the medical system 100, such as temperature, delivered voltage, or the like, and for measuring and monitoring one or more tissue characteristics, such as EGM waveforms, monophasic action potentials, tissue impedance, or the like, in addition to monitoring, recording, or otherwise conveying measurements or conditions within the energy delivery device or other component of system 100 or the ambient environment at the distal portion of the energy delivery device. The sensor(s) may be in communication with controller 104 for initiating or triggering one or more alerts or ablation energy delivery modifications during operation of the energy delivery device. In some examples, such sensors may be part of controller 104, robotic delivery system 116, and/or anatomical information device(s) 114.

In some examples, system 100 may include catheter stabilization techniques that may be employed during energy delivery to maintain a position of catheter 102 while energy delivery elements 110 may be disconnected from an electrophysiology recording device (which in some examples may be part of controller 200) and electric potential based navigation systems (e.g., of anatomical information device(s) 114). For example, system 100 may deploy a structure (not shown) which may be configured to stabilize catheter 102 in place. In addition, it may be desirable for the system to briefly disconnect other positioning and navigation elements including electromagnetic navigation coils from the navigation processor during high voltage therapeutic energy delivery pulses to avoid distortions in positional stability.

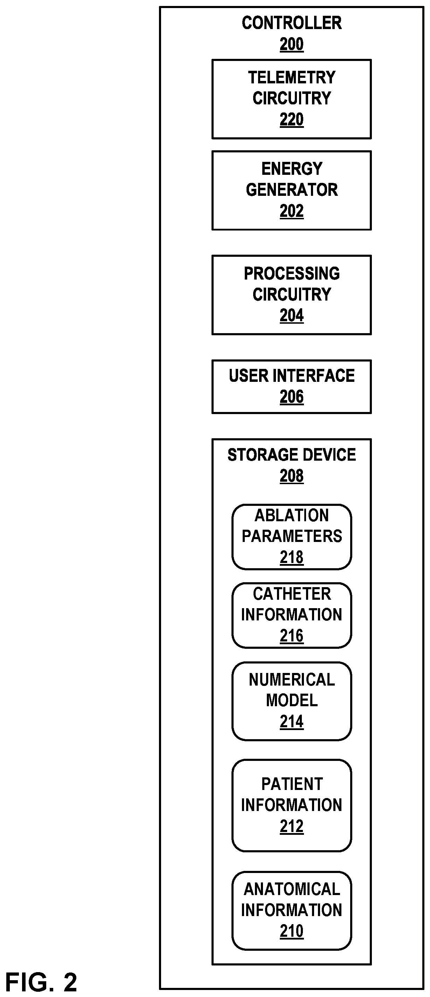

FIG. 2 is a block diagram illustrating an example controller of an ablation system, in accordance with one or more aspects of this disclosure. Controller 200 of FIG. 2 may be an example of controller 104 of FIG. 1. As shown in FIG. 2, controller 200 may include energy generator 202, processing circuitry 204, user interface 206, and storage device 208.

Energy generator 202 may be configured to control energy delivery elements 110 of catheter 102 (FIG. 1) such as to provide electrical pulses to electrodes (e.g., energy delivery elements 110) to perform an electroporation procedure or other ablation procedure to cardiac tissue or other tissues within the patient's body, including but not limited to renal tissue, airway tissue, bones, organs, or tissue within the cardiac space or the pericardial space. For instance, energy generator 202 may be configured and programmed to deliver pulsed, high-voltage electric fields appropriate for achieving desired pulsed, high-voltage ablation (referred to as "pulsed field ablation" or "pulsed electric field ablation") and/or pulsed radiofrequency ablation. In another example, energy generator 202 may be configured to control one or more cryogenic energy delivery elements to achieve desired cryogenic ablation. While shown in the example of FIG. 2 as a single energy generator, energy generator 202 is not so limited. For instance, controller 200 may include multiple energy generators that are each capable of generating ablation signals in parallel. In some examples, controller 200 may include energy generators of different types, such as a pulsed field energy generator, a radio frequency energy generator, and/or a cryogenic energy generator.

Processing circuitry 204 may include one or more processors, such as any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 210 herein may be embodied as firmware, hardware, software or any combination thereof. Processing circuitry 204 controls energy generator 202 to generate signals according to various settings (e.g., linear settings 430 or focal settings 432). In some examples, processing circuitry 204 may execute other instructions stored in storage device 208 to perform ablation based at least in part on anatomical information 210.

Storage device 208 may be configured to store information within controller 200, respectively, during operation. Storage device 208 may include a computer-readable storage medium or computer-readable storage device. In some examples, storage device 208 includes one or more of a short-term memory or a long-term memory. Storage device 208 may include, for example, random-access memories (RAM), dynamic random-access memories (DRAM), static random-access memories (SRAM), ferroelectric random-access memories (FRAM), magnetic discs, optical discs, flash memories, or forms of electrically programmable memories (EPROM) or electrically erasable and programmable memories (EEPROM). In some examples, storage device 208 is used to store data indicative of instructions, e.g., for execution by processing circuitry 204, respectively. Storage device 208 may be configured to store anatomical information 210, patient information 212, numerical model 214, catheter information 216, and/or ablation parameters 218.

Anatomical information 210 may be patient specific anatomical information, typical patients' anatomies, and/or a combination of the two, enabling augmentation of the patient specific anatomical information. For example, one patient may have a different anatomy than another patient. Anatomical information 210 may be generated by a computed tomography (CT) device, a magnetic resonance imaging (MRI) device, an ultrasound (U/S) device, or from electrical characterization, such as from a pacing device, an electrophysiology (EP) mapping device, and/or a non-invasive mapping device (which may be examples of anatomical information device(s) 114). In some examples, storage device 208 may also store patient information 212 such as the patient history which may include comorbidities, prior ablation strategies and procedures, electrocardiogram (ECG) information (e.g., from a 12-lead ECG device), or device information from implantable pulse generators (IPGs), implantable cardiac defibrillators (ICDs), and/or implantable loop recorders. For example, patient information 212 may include physiological information, such as that sensed by an IPG or an ICD. Patient information 212 may include physiological information including cardiac electrophysiological information. Catheter information 216 may include geometry information of catheter 102, such as size, location of energy delivery elements 110, etc.

User interface 206 may include a button or keypad, lights, a speaker for voice commands, a display, such as a liquid crystal (LCD), light-emitting diode (LED), or organic light-emitting diode (OLED). In some examples, the display may be configured to display a representation of a suggested positioning of at least one energy delivery element of energy delivery elements 110. For example, processing circuitry 204 may load numerical model 214 and use numerical model 214 to determine, based on anatomical information 210 and/or patient information 212, suggested target tissue for ablation.

The display may be used to guide the clinician in positioning the at least one energy delivery element to a position in contact with or near the suggested target tissue such that during the delivery of energy to the at least one energy delivery element, the target tissue is ablated. In some examples, the display may display a representation of the target tissue that will be ablated after delivery of ablation energy via the at least one energy delivery element. For example, the display may display the target tissue differently than surrounding tissue. For example, the display may display the target tissue in a different color, with different shading, or with different patterned fill, than surrounding tissue. In some examples, the display may display, after the delivery of ablation energy via the at least one energy delivery element, a representation of the tissue that was ablated. For example, processing circuitry 204 may determine which tissue was ablated, through, for example, new anatomical information generated by anatomical information device(s) 114, patient information 212, or by sensors of system 102. Processing circuitry 204 may then output for display to user interface 206 a representation of the tissue that was ablated. In some examples, the display includes a touch screen. User interface 206 may be configured to display any information related to the performance of ablation. User interface 206 may also receive user input (e.g., selection of target tissue) via user interface 206. The user input may be, for example, in the form of pressing a button(s) on a keypad or selecting an icon(s) from a touch screen.

In some examples, user interface 206 may be configured to allow a clinician to select target tissue for ablation. For example, user interface 206 may display a representation of cardiac tissue in a region of interest. User interface 206 may permit the clinician to select the target tissue by tapping, circling, or otherwise indicating on user interface 206 the tissue the clinician would like ablated. In such examples, processing circuitry 204 may determine, using numerical model 214, anatomical information 210, and/or patient information 212, ablation parameters 218 for use to ablate the target tissue. Such ablation parameters 218 may include a suggested one or more energy delivery elements for delivering energy to the target tissue, a suggested positioning for one or more energy delivery elements 110, a suggested energy level to be delivered, an energy modality (e.g., RF, cryogenic, PFA, etc.) or combination of modalities, or the like. For clarity, the delivery elements 110 may reside on separate catheters 102 placed in different anatomic locations, including inside the cardiac chambers, in the pericardial space, on the body surface, or in other anatomic locations enabling preferential energy delivery.

The effect on target tissue of energy delivered through one or more energy delivery elements may be relatively accurately predicted using numerical model 214, for example using Maxwell's equations. In some examples, numerical model 214 may be a machine learning model or an artificial intelligence model. Such a model may be trained using anatomical information 210, patient information 212, and/or catheter information 216. For example, with pulsed field ablation, the electric field effect created by short electric field applications between indwelling energy delivery elements, such as bipole(s), may be relatively easily calculable and combined with ablation parameters 218 such as an energy level to be used, a number of trains or pulses with pulsed field ablation, the energy delivery element(s) to be used, to identify real affected target tissue geometries instead of simple point registration of where an electrode was during some portion of delivery of ablation therapy. Because the field effect is fast and calculable, a robust assessment of the affected areas may be possible.

In some examples, processing circuitry 204 may use anatomical information 210, patient information 212, and/or catheter information 216 to, for example, through numerical modeling using numeric model 214, to propose lesion locations (e.g., target tissue for ablation), select targeted regions, simulate lesion sets to impact the arrythmia. For example, processing circuitry 204 may select or specify energy generator 202 and catheter 102 capabilities of performing/creating lesions, as well as suggest energy information for one of more of energy delivery elements 110 on catheter 102 (both of FIG. 1). Clinicians may generate their ablation plan based on suggested information displayed on user interface 206 or override the suggested information based on their medical judgement. After the physician navigates the ablation catheter to the target tissue manually (or alternatively using robotic delivery system 116), processing circuitry 204 may detect new inputs from anatomical information device(s) 114, such as catheter location, and proximity to the target tissue. The algorithm may then iterate numeric model 214 on energy delivery element combinations, sizes, locations, and/or spacing. Processing circuitry 204 may control one or more of energy delivery elements 110 to deliver therapy and, in the case of a pulsed field ablation catheter, modulate energy delivery during delivery of therapy to achieve a desired outcome. Such modulation of energy delivery may include pulse train parameters, such as pulse waveform morphology (e.g. square wave, triangle wave, sine wave), pulse duration (e.g., pulse width), pulse amplitude (e.g., voltage), number of pulses in a train, inter-pulse delay (e.g., time between each pulse), amplitude modulation over the course of a train, pulse duration modulation over the course of a train, as well as controlling the optimal inter-electrode electric field vectoring schemes to optimize desired therapeutic effect). In some examples, processing circuitry 204 may compare the effect of the ablation to the prediction as each lesion is created. For example, processing circuitry 204 may control one or more of energy delivery elements to deliver energy to target tissue and determine an effect of the delivery of the energy to the target tissue. For example, processing circuitry may use information from anatomical information device(s) 114 to determine a size, shape, and location of a lesion. Processing circuitry 204 may determine if the treatment was either effective or needed modification based on a comparison of the information regarding the lesion and the predicted effect of the ablation. In some examples, processing circuitry 204 may update or train numerical model 214 based on the comparison.

For example, processing circuitry 204 may create a patient-specific numerical model 214 which may be trained on or derived from pre-procedural and/or intra-procedural anatomical information and/or electroanatomical information, and store such information as anatomical information 210. Processor circuitry 204 may determine ablation target tissue to lessen, terminate or cure arrhythmia. In some examples, the target tissue may be directly extracted from a mapping system of anatomical information device(s) 114, for example atrial fibrillation drivers determined by a mapping system, such as CardioInsight^{™} or Affera^{™} Mapping and Ablation System by Medtronic plc of Dublin, Ireland, or as specified by the clinician, for example, via user interface. Anatomical information related to the target tissue and/or surrounding tissue may be stored in anatomical information 210.

Processing circuitry 204 may determine ablation parameters 218 based at least in part on anatomical information 210. For example, processing circuitry 204 use numerical model 214 to determine ablation parameters 218 based on anatomical information 210, patient information 212, and/or catheter information. Anatomical information 210 may include target tissue thickness, target tissue condition, surrounded structures, and/or the like. As an example, real-time, non-contact ultrasound measurements of anatomical information device(s) 114 may be used by processing circuitry 204 to measure local tissue thickness. The relationship between the catheter-based energy delivery elements and the target tissue can be determined either by an electroanatomic mapping and navigation system, through medical imaging (for example, the same ultrasound system used to measure tissue thickness), or the like.

Once the target tissue has been determined, processing circuitry 204 may determine predictions, prescribe the appropriate positioning of at least one of energy delivery elements 110, and/or determine the level of energy to be delivered to create the lesion morphology that was either prescribed by the processing circuitry 204 or input through user interface 206 by a clinician. For example, anatomical information device(s) 114 may communicate with controller 200 via telemetry circuitry 220. Processing circuitry 204 may use numerical model 214 and information from anatomical information device(s) 114 to adjust the ablation field size in accordance with the distance of the catheter to the heart wall, targeted lesion depth, lesion morphology and energy delivery element locations. Processing circuitry 204 may output for display, on user interface 206 or other display, a representation of a proposed location to achieve the desired lesion morphology. For example, user interface 206 may display a shadow(s), color(s), patterned fill, or the like, to direct the position of the at least one energy delivery element to a more ideal location to achieve the desired lesion morphology. In some examples, processing circuitry 204 may control robotic delivery system 116 to automatically move catheter 102 to an idealized or preferred location or set of idealized or preferred locations determined by processing circuitry 204 using numerical model 214 and catheter information 216. In addition, processing circuitry 204 may output for display on user interface 206 a volume of tissue that will be ablated given ablation parameters 218 to enable the clinician to compare the anticipated ablation volume to the desired tissue target. In some examples, the tissue that will be ablated may be displayed using a different color(s), shading(s), patterned fill, or the like, than surrounding tissue.

For example, user interface 206 or another display, may depict a representation of the predicted tissue volume or geometry to be ablated from the one or more energy delivery elements 110. Processing circuitry 204 may determine the prediction using numerical model 214. Numerical model 214 may base such a prediction on estimated tissue contact and proximity, electric field modeling, energy vectoring scheme chosen by the clinician or determined by processing circuitry 204, the energy level and other ablation parameters (e.g., of ablation parameters 218) selected by the clinician or determined by processing circuitry 204. By incorporation of anatomical information 210, including the tissue thickness in the region of interest, may predict the lesion depth or geometry into the target tissue. When delivering pulsed field ablation energy, such deliveries between one or more electrodes on one energy delivery catheter or between one or more electrodes on more than one catheter may be vectored between differing electrodes, thus vectoring the electric fields in more than one direction. Such multiple electrode energy vectoring schemes may be optimally determined by the numerical model and executed by the processor circuitry.

In some examples, processing circuitry 204 may output for display to user interface 206, or another display, the predicted lesion volume and location. In this manner, a clinician may input through user interface 206 a selection of which of energy delivery elements 110 to use to deliver ablation therapy or other ablation parameters (e.g., of ablation parameters 218) and view the predicted lesion volume and location based on the selected ablation parameters. Thus, such a predictive ability allows the clinician to select or deselect specific energy delivery elements 110 to achieve the desired prediction of lesion extent to treat the arrhythmia or other cardiac disease condition projected onto the anatomical rendering. In some examples, after energy delivery, processing circuitry 204 may use, for example, multi-frequency impedance magnitude and phase angle post-delivery to estimate the area and volume of ablated tissue, which may then be projected onto the anatomical rendering and displayed for the clinician. Sub-ablation threshold energy may also be used to confirm circuit integrity and to measure fields generated on the various energy delivery elements 110 and/or any other electrodes residing in or on the patient.

Following ablation, processing circuitry 204 may output for display, on user interface 206 or other display, visual indicators of metrics associated with the created lesion. For example, a compilation of displayed lesions may be displayed that indicate lesion depth and morphology of previously delivered lesions. Processing circuitry 204 may also output for display a visual indication of where there might not be completely transmural sections of the lesions such that the clinician might reposition the catheter to complete the lesion set desired. Processing circuitry 204 may also update anatomical information 210 to remove the ablated tissue from anatomical information 210. Processing circuitry 204 may also update numerical model 214 based upon new information gained on the anatomy (e.g., if a different amount of tissue was ablated than was predicted) or cardiac rhythms. In some examples, processing circuitry 204 may confirm success of the ablation procedure or to determine new target tissue for ablation based on updated anatomical information 210, patient information 212, and/or the updated numerical model 214.

For example, processing circuitry 204 may output for display, on user interface 206 or other display, a representation of the estimated tissue volume that was ablated, both at that location and adjusted for any changes in the location of energy delivery element(s) during energy delivery, as well as based on estimated tissue contact and proximity, electric field modeling, energy vectoring scheme chosen by the clinician or determined by processing circuitry 204, and the energy level and any other ablation parameters of ablation parameters 218 selected by the clinician or determined by processing circuitry 204.

Telemetry circuitry 220 may include any suitable hardware, firmware, software or any combination thereof for communicating with another device, such as anatomical information device(s) 114 or robotic delivery system 116. Telemetry circuitry 220 may be configured to communicate using any of a variety of wireless communication schemes, such as Bluetooth^{®} or Bluetooth Low Energy^{®}, WiFi, 4G, or 5G, or a wired communication scheme such as ethernet. Under the control of processing circuitry 204, telemetry circuitry 220 may receive downlink telemetry from, and/or send uplink telemetry to, robotic delivery system 116 or anatomical information device(s) 114 with the aid of an internal or external antenna, or via wires

In some examples, processing circuitry 204 may use a differential impedance amplitude and phase angle (DIAPA) measurement to estimate the relative myocardial/blood contact ratio or quality of myocardial contact. In some examples, processing circuitry 204 may use the estimated quality of myocardial contact to adjust for position shift in an electric potential based navigation system (e.g., of anatomical information devices 114). In some examples, controller 200 may include an electric potential or electromagnetic based navigation system. In such cases, controller 200 may use measurements to improve navigation positioning accuracy and precision, before, during, and after the delivery of ablation energy. Such measurements and factors to be utilized to ensure that the catheter position on user interface 206 is accurately depicted and not significantly altered as a result of energy delivery. These measurements may include the quality of myocardial contact based on DIAPA, a predicted lesion geometry, and pre and post energy delivery measurements of tissue conductivity or impedance to quantify the reduction in myocardial impedance detected in the local myocardium as a result of the energy delivery. In the example of pulsed field ablation, the predicted lesion geometry may be determined as an estimated extent of the Irreversible Electroporation (IRE) isopotential surface in the environment surrounding energy delivery elements 110, based on estimated tissue contact and proximity, electric field modeling, energy vectoring scheme chosen by the clinician (or determined by processing circuitry 204), and the amplitude and pulsed field ablation pulse parameters selected by the clinician.

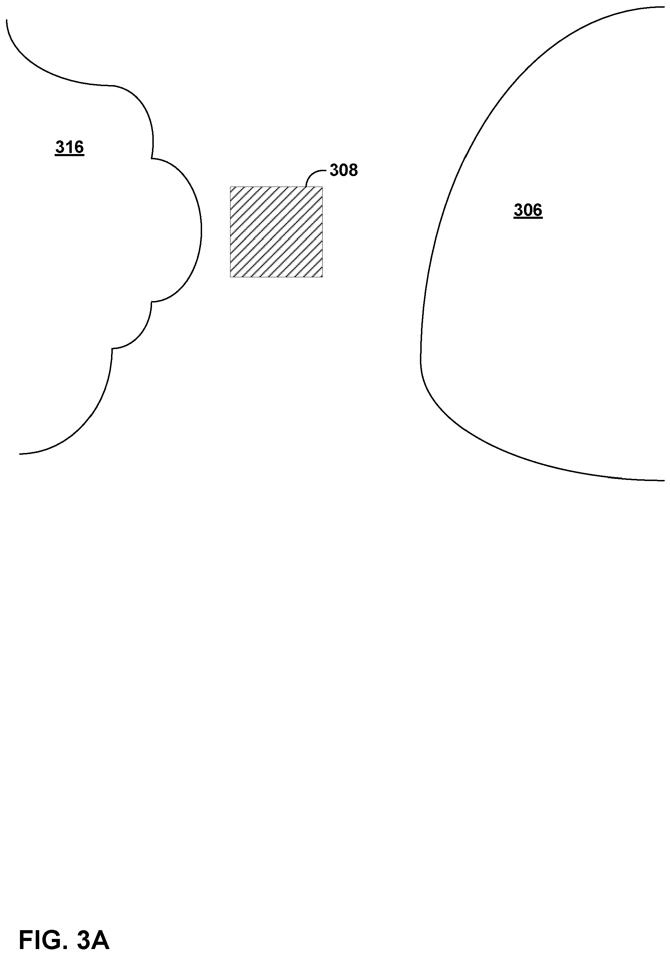

FIGS. 3A-3D are conceptual diagrams illustrating examples of information displayed by a user interface in accordance with one or more techniques of this disclosure. FIG. 3A may be an example of information displayed by user interface 206 prior to delivery of an ablation therapy. In the example of FIG. 3A, processing circuitry 204 may generate and output for display to a user interface, such as user interface 206, a representation of a suggested positioning of at least one energy delivery element 308, a representation of tissue 316, and a representation of tissue 306. User interface 206 may display representation of a suggested positioning of at least one energy delivery element 308, representation of tissue 316, and representation of tissue 306. By displaying representation of suggested positioning of at least one energy delivery element 308, controller 200 (FIG. 2) may guide a clinician that may be manually inserting catheter 102 (FIG. 1) into the anatomy of a patient. When catheter 102 reaches an area within the anatomy represented on user interface 206, processing circuitry 204 may generate and output for display a representation of catheter 102 (not shown in FIG. 3A). Additionally, when one or more energy delivery elements 110 reach the suggested positioning within the actual anatomy of the patient, processing circuitry 204 may control user interface 206 to issue an alert. For example, processing circuitry 204 may issue an alert when the at least one energy delivery element of energy delivery elements 110 is within a treatment range (e.g., within suggested positioning of at least one energy delivery element 308) of the target tissue. For example, processing circuitry 204 may change the manner in which user interface 206 may display the representation of suggested positioning of at least one energy delivery element 308. For example, user interface 206 may flash, change the color, change the geometric fill, or remove representation of a suggested positioning of at least one energy delivery element 308. In some examples, user interface 206 may output an audible alarm when the at least one energy delivery element is within the treatment range. In some examples, processing circuitry 204 may control the at least one energy delivery element to automatically begin delivering energy when the at least one energy delivery element is within the treatment range of the target tissue.

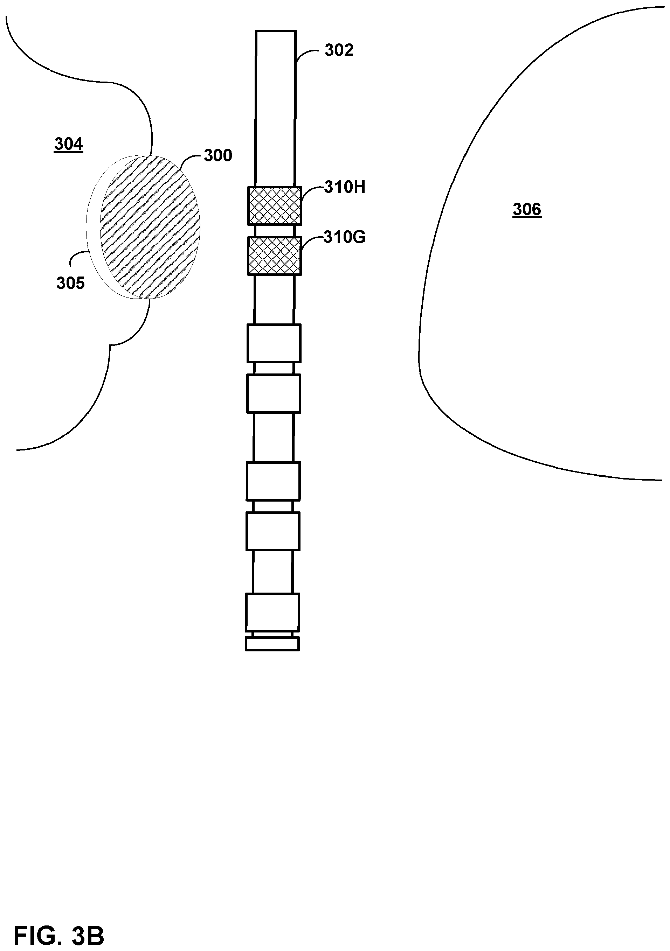

FIG. 3B may be an example of information displayed by user interface 206 prior to delivery of an ablation therapy, but after guidance of one or more energy delivery elements 110 of catheter 102 to representation of a suggested positioning of at least one energy delivery element 308. For example, processing circuitry 204 may generate and output for display a representation of a catheter 302, a representation of target tissue to be ablated 300, a representation of predicted tissue that will be ablated 305, a representation of surrounding tissue 304, and representation of tissue 306. In some examples, both the tissue to be ablated 300 and predicted tissue that will be ablated 305 may both be displayed. In some examples, the tissue to be ablated 300 and predicted tissue that will be ablated 305 may be the same (e.g., tissue to be ablated 300). In other examples, tissue to be ablated 300 may be different than predicted tissue that will be ablated 305, as shown. In some examples, user interface 206 may display energy delivery element(s) in a manner that distinguishes energy delivery element(s) that are selected to deliver ablation therapy from other energy delivery element(s). For example, if catheter 102 of FIG. 1 is the catheter being represented in representation of a catheter 302 and energy delivery elements 110G and 110H are selected to deliver energy, user interface 206 may display representations of energy delivery elements 310G and 310H, which correspond to energy delivery elements 110G and 110H, in a different manner than unselected energy delivery elements. For example, user interface 206 may display the selected energy delivery elements 310G and 310H using a different color, different shading, different pattern fill, flashing, or the like to inform the clinician of which energy delivery elements are currently selected.

User interface 206 may also similarly identify target tissue to be ablated 300 using a different color, different shading, different pattern fill, flashing, or the like than representation of surrounding tissue 304 or representation of tissue 306 to inform the clinician of the location and geometry of the lesion to be created by the delivery of ablation energy. If the clinician enters a new selection of energy delivery element(s), user interface 206 may change the visual indication of the energy delivery element(s) such that the newly selected energy delivery element(s) are visibly identifiable. Similarly, the target tissue to be ablated may change based on the newly selected energy delivery element(s). In such a case, user interface 206 may change the visual indication of the new target tissue to be visibly identifiable. For example, user interface 206 may change the visual representation of energy delivery elements 310G and 310H to match the previously unselected energy delivery elements (e.g., to be white) and target tissue to be ablated 300 to match surrounding tissue 304 (e.g., to be white). User interface 206 may also change the visual representation of the newly selected energy delivery elements (e.g., to be pattern filled), which may be on one or more catheters, such as catheter 102, and update the location and geometry of the new target tissue to be ablated (e.g., to be pattern filled). In this manner, controller 200 may provide to a clinician information related to which tissue will be ablated, which may improve client outcomes.

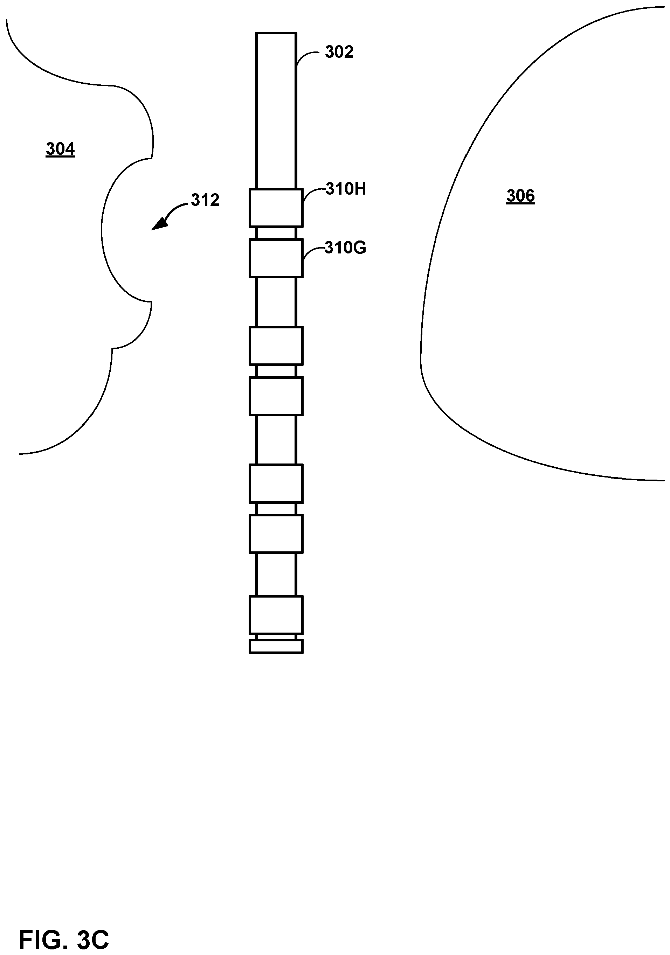

FIG. 3C may be an example of information that is displayed by user interface 206 after lesion 312 is created. For example, user interface 206 may change the representation of energy delivery elements 310G and 310H as they may no longer be selected. In some examples, user interface 206 may only change the representation of energy delivery elements 310G and 310H upon receipt of a command from a clinician to deselect such energy delivery elements. In some examples, user interface 206 may display surrounding tissue 304 and tissue 306. As target tissue to be ablated 300 (FIG. 3B) has been ablated, user interface 206 may not display target tissue to be ablated 300, but instead display lesion 312, and/or may represent the target tissue to be ablated which has now been ablated, by being exposed to pulsed electric fields that have exceeded a threshold to irreversibly electroporate said tissues (to create lesions), in a different manner than before the delivery of energy (e.g., in a different color). Additionally, or alternatively, user interface 206 may display or depict in an appropriate manner, areas or volumes of tissue which have been exposed to repeated or overlapping deliveries, all (or a plurality) of which exceeded the minimum threshold for lesion creation. Such overlapping energy deliveries ensure that gaps are not created between ablated tissues. User interface 206's depiction of number of overlapping or repeated exposures to threshold pulsed electric fields may include using a variety of graphical approaches, such as variations in colors or intensity. In this manner, a clinician may strive to produce overlap around all of the tissues targeted for contiguous ablation.

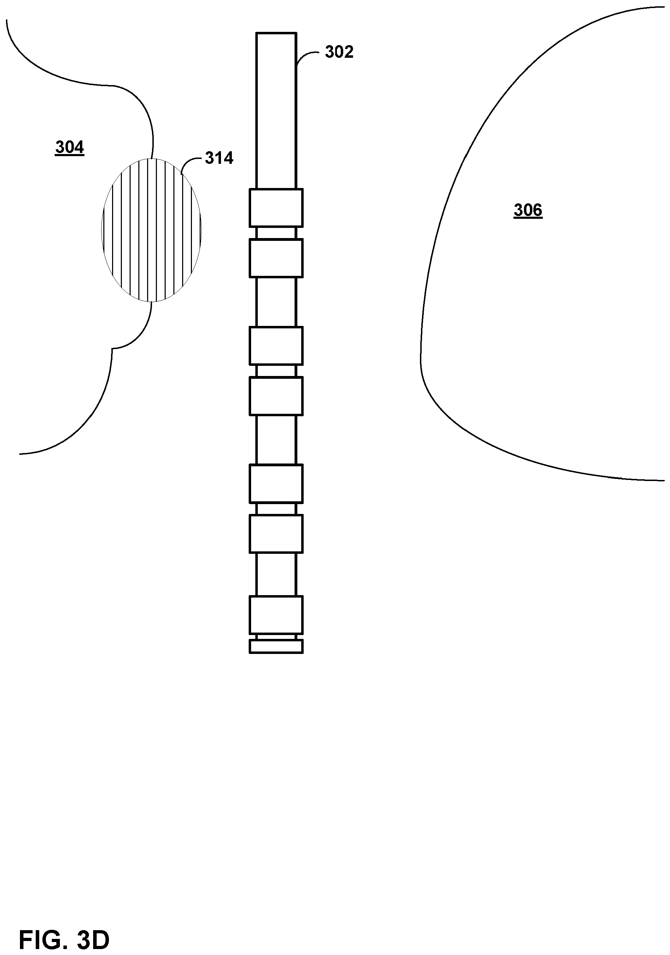

FIG. 3D is another example of information that may be displayed by user interface 206 after a lesion is created. In the example of FIG. 3D, rather than display lesion 312, user interface 206 may display a representation of tissue that was ablated 314 in a different manner than representation of target tissue to be ablated 300 of FIG. 3B. For example, user interface 206 may change the color, shading, pattern fill, or flash the representation, or the like to inform the clinician of that the tissue has been ablated.

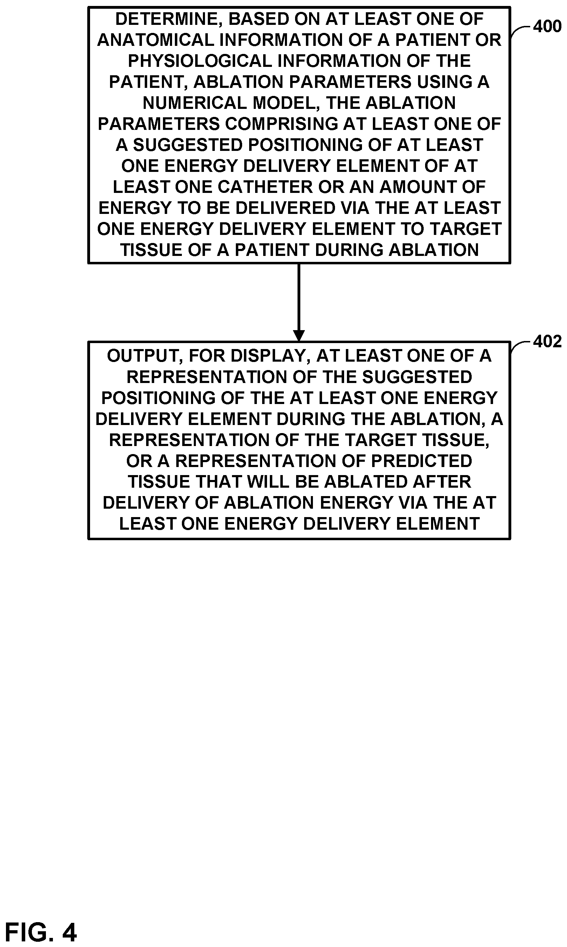

FIG. 4 is a flowchart illustrating an example ablation assistance technique in accordance with one or more techniques of this disclosure. The technique of FIG. 4 may be performed by a controller, such as controller 104 of FIG. 1 or controller 200 of FIG. 2. Controller 200 may determine, based on at least one of anatomical information of a patient or physiological information of the patient, ablation parameters 218 using a numerical model, the ablation parameters 218 comprising at least one of a suggested positioning of at least one energy delivery element of at least one catheter or an amount of energy to be delivered via the at least one energy delivery element to target tissue of the patient during ablation (400). For example, processing circuitry 204 of controller 200 may load numerical model 214 and use numerical model 214, anatomical information 210 and/or patient information 212 to determine ablation parameters 218. Ablation parameters 218 may include the suggested positioning of, for example, energy delivery elements 110G and 110H, and an amount of energy to be delivered via energy delivery elements 110G and 110H. Target tissue 300 (FIG. 3B) may include an area or volume of tissue to be ablated via delivery of pulsed electrical fields that exceed a threshold to irreversibly electroporate the target tissue.

Controller 200 may output, for display, at least one of a representation of the suggested positioning of the at least one energy delivery element during the ablation, a representation of the target tissue, or a representation of predicted tissue that will be ablated after delivery of ablation energy via the at least one energy delivery element (402). For example, controller 200 may output for display suggested positioning 308 (FIG. 3A) near the target tissue to be ablated. Alternatively, or additionally, controller 200 may output, for display, a representation of the target tissue to be ablated 300 and/or predicted tissue that will be ablated 308 (FIG 3B). In some examples, controller 200 outputs such representation(s) to user interface 206 and user interface 206 displays such representation(s).

In some examples, numerical model 214 is an artificial intelligence model. For example, numerical model 214 may be a rule-based model. In such an example, numerical model 214 may be programmed to include rules by which numerical model 214 operates when executed by processing circuitry 204. In some examples, numerical model 214 is a machine learning model. The machine learning model may be trained, for example, on anatomical information, physiological information (which may include electrophysiological information), patient information, energy delivered during ablation, and/or tissue that was ablated during ablation for a plurality of patients. Potential machine learning models may include a k-means clustering model, Naive Bayes, k-nearest neighbors, random forest, support vector machines, neural networks, linear regression, logistic regression, etc.

In some examples, controller 200 may, after the delivery of ablation energy via the at least one energy delivery element, determine a tissue that was ablated and output for display a representation of the tissue that was ablated. In some examples, controller 200 may, after the delivery of ablation energy via the at least one energy delivery element, determine a tissue that was ablated and output for display a representation of the surrounding tissue that does not comprise the tissue that was ablated. For example, controller 200 may output for display a representation of surrounding tissue, the surrounding tissue including tissue adjacent to the tissue that was ablated and not including the tissue that was ablated. In some examples, controller 200 may update the stored anatomical information (e.g., anatomical information 210) based on the determination of the tissue that was ablated. In some examples, controller 200 may modulate the energy delivery in real-time to create the desired lesion.

In some examples, the at least one catheter (e.g., catheter 100) comprises at least one of a pulsed field ablation catheter, a cryoablation catheter, a laser ablation catheter, a thermal ablation catheter, radioablation catheter, an ultrasonic ablation catheter, a thermal ablation catheter, a carbon ion beam catheter, a radio frequency ablation catheter, a microwave ablation catheter, or the like. In some examples, controller 200 may control delivery of the at least one catheter into an anatomy of the patient via robotics towards the target tissue in response to receiving a user input of a selection of the target tissue, outputting, by the processing circuitry, for display the representation of the target tissue that will be ablated after delivery of ablation energy via the at least one energy delivery element (e.g., target tissue to be ablated 300).

In some examples, controller 200 may receive the anatomical information 210 or the physiological information from one or more of a computed tomography device, a magnetic resonance imaging device, an ultrasound device, a pacing device, an electrophysiology mapping device, or a non-invasive mapping device. In some examples, controller 200 may determine a distance from the at least one energy delivery element to the target tissue, wherein the energy to be delivered to the target tissue during ablation is further based on the distance from the at least one energy delivery element to the target tissue. In some examples, controller 200 may issue an alert when the at least one energy delivery element is within a treatment range of the target tissue. In some examples, controller 200 may automatically begin delivering energy via the at least one energy delivery element when the at least one energy delivery element is within the treatment range of the target tissue.

In some examples, controller 200 may control the at least one energy delivery element during delivery of ablation energy to adjust ablation parameters 218. In some examples, controller 200 may control the at least one energy delivery element to deliver non-therapeutic ablative energy, and test, based on the delivery of the non-therapeutic energy, assumptions in numerical model 214. For example, controller 200 may sense (e.g., via one or more of energy delivery elements 110 or sensors), a physiological response to the delivery of non-therapeutic energy and compare the sensed physiological response to a predicted physiological response to test the assumptions in numerical model 214. If the physiological response is outside of a predetermine range of the predicated physiological response, controller 200 may determine at least one assumption in numerical model 214 is incorrect and may update or train numerical model 214 to improve the accuracy of numerical model 214.

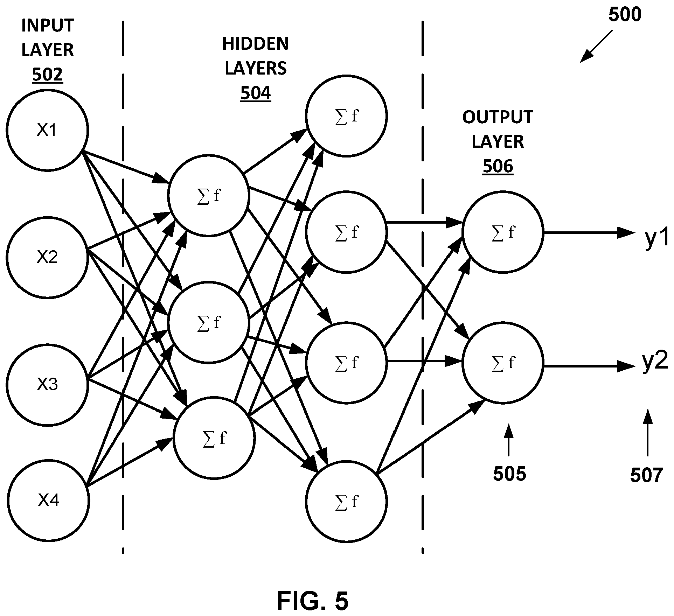

FIG. 5 is a conceptual diagram illustrating an example machine learning model according to one or more techniques of this disclosure. Machine learning model 500 may be an example of the numerical model 214 of FIG. 2. Machine learning model 500 may be an example of a deep learning model, or deep learning algorithm, trained to determine ablation parameters 218. Controller 200 may train, store, and/or utilize machine learning model 500. In some examples, other types of machine learning and deep learning models or algorithms may be utilized in other examples. For example, a convolutional neural network model of ResNet-18 may be used. Some non-limiting examples of models that may be used for transfer learning include AlexNet, VGGNet, GoogleNet, ResNet50, or DenseNet, etc. Some non-limiting examples of machine learning techniques include Support Vector Machines, K-Nearest Neighbor algorithm, and Multi-layer Perceptron.

As shown in the example of FIG. 5, machine learning model 500 may include three types of layers. These three types of layers include input layer 502, hidden layers 504, and output layer 506. Output layer 506 comprises the output from the transfer function 505 of output layer 506. Input layer 502 represents each of the input values X1 through X4 provided to machine learning model 500. In some examples, the input values may include any of the of values input into the machine learning model, as described above. For example, the input values may include anatomical information 210, patient information 212, catheter information 216, and/or other data as described above. In addition, in some examples input values of machine learning model 500 may include additional data, such as other data that may be collected by or stored in controller 200 (or any other device or system described herein).

Each of the input values for each node in the input layer 502 is provided to each node of a first layer of hidden layers 504. In the example of FIG. 5, hidden layers 504 include two layers, one layer having four nodes and the other layer having three nodes, but fewer or greater number of nodes may be used in other examples. Each input from input layer 502 is multiplied by a weight and then summed at each node of hidden layers 504. During training of machine learning model 500, the weights for each input are adjusted to establish the relationship between anatomical information 210, patient information 212, catheter information 216 and ablation parameters 218. In some examples, one hidden layer may be incorporated into machine learning model 500, or three or more hidden layers may be incorporated into machine learning model 500, where each layer includes the same or different number of nodes.

The result of each node within hidden layers 504 is applied to the transfer function of output layer 506. The transfer function may be liner or non-linear, depending on the number of layers within machine learning model 500. Example non-linear transfer functions may be a sigmoid function or a rectifier function. The output 507 of the transfer function may be one or more classification that anatomical information 210, patient information 212, catheter information 216 are indicative of particular ablation parameters of ablation parameters 218, and/or the like.

As shown in the example above, by applying machine learning model 500 to input data such as anatomical information 210, patient information 212, and/or catheter information 216 processing circuitry 204 is able to determine ablation parameters 218. This may improve patient outcomes.

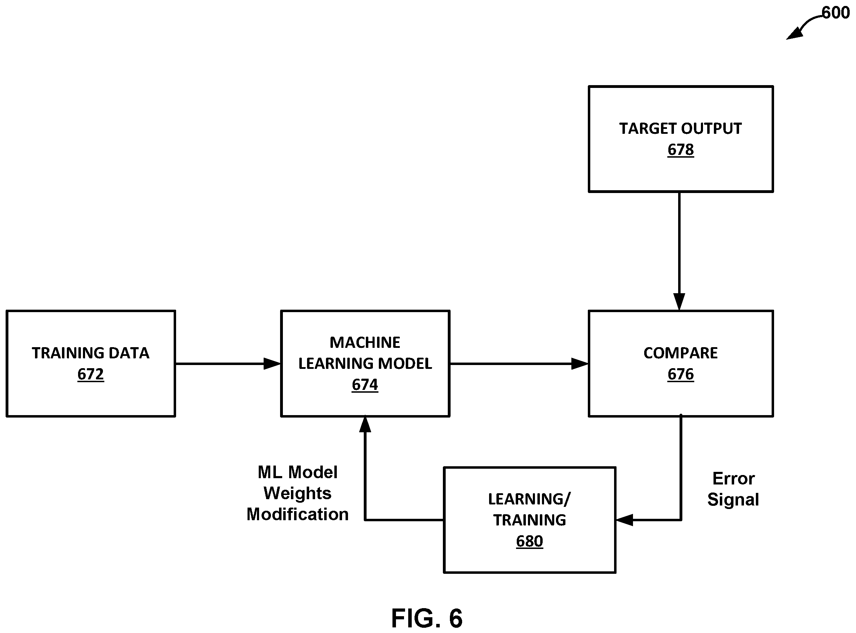

FIG. 6 is a conceptual diagram illustrating an example training process for a machine learning model according to one or more techniques of this disclosure. Process 600 may be used to train machine learning model 500 and/or numerical model 214. A machine learning model 674 (which may be an example of machine learning model 500 and/or numerical model 214) may be implemented using any number of models for supervised and/or reinforcement learning, such as but not limited to, an artificial neural network, a decision tree, naive Bayes network, support vector machine, or k-nearest neighbor model, CNN, RNN, LSTM, ensemble network, to name only a few examples. In some examples, controller 200 and/or controller 104 initially trains machine learning model 674 based on a corpus of training data 672. Training data 672 may include, for example, anatomical information 210, patient information 212, catheter information 216, ablation parameters 218, physiological information, energy delivered during ablation, tissue that was ablated during ablation, pre-procedural, intra-procedural anatomical information, electroanatomical information, a comparison of information regarding a lesion and a predicted effect of an ablation, and/or any other training data described herein.

While training machine learning model 674, processing circuitry 204 may compare 676 a prediction or classification with a target output 678. Processing circuitry 204 may utilize an error signal from the comparison to train (learning/training 680) machine learning model 674. Processing circuitry 204 may generate machine learning model weights or other modifications which processing circuitry 204 may use to modify machine learning model 674. For example, processing circuitry 204 may modify the weights of machine learning model 500 based on the learning/training 680. For example, controller 200 and/or controller 104 may, for each training instance in training data 672, modify, based on training data 672, the manner in which ablation parameters 218 are determined.

The techniques described in this disclosure may be implemented, at least in part, in hardware, software, firmware or any combination thereof. For example, various aspects of the described techniques may be implemented within processing circuitry, which may include one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit including hardware may also form one or more processors or processing circuitry configured to perform one or more of the techniques of this disclosure.

Such hardware, software, and firmware may be implemented, and various operation may be performed within same device, within separate devices, and/or on a coordinated basis within, among or across several devices, to support the various operations and functions described in this disclosure. In addition, any of the described units, circuits or components may be implemented together or separately as discrete but interoperable logic devices. Depiction of different features as circuits or units is intended to highlight different functional aspects and does not necessarily imply that such circuits or units must be realized by separate hardware or software components. Rather, functionality associated with one or more circuits or units may be performed by separate hardware or software components or integrated within common or separate hardware or software components. Processing circuitry described in this disclosure, including a processor or multiple processors, may be implemented, in various examples, as fixed-function circuits, programmable circuits, or a combination thereof. Fixed-function circuits refer to circuits that provide particular functionality with preset operations. Programmable circuits refer to circuits that can be programmed to perform various tasks and provide flexible functionality in the operations that can be performed. For instance, programmable circuits may execute software or firmware that cause the programmable circuits to operate in the manner defined by instructions of the software or firmware. Fixed-function circuits may execute software instructions (e.g., to receive stimulation parameters or output stimulation parameters), but the types of operations that the fixed-function circuits perform are generally immutable. In some examples, one or more of the units may be distinct circuit blocks (fixed-function or programmable), and in some examples, one or more of the units may be integrated circuits.

The techniques described in this disclosure may also be embodied or encoded in a computer-readable medium, such as a computer-readable storage medium, containing instructions that may be described as non-transitory media. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

For simplicity, examples provided in this disclosure have focused mainly on two-dimensional catheter and tissue representations. It should be understood that this disclosure applies similarly to more complex and three-dimensional catheter constructs, such as electrode arrays mounted on a plurality of expandable splines, on a deformable or on the expandable spherical mesh of wire matrix, or on an expandable balloon or on the structure of a curvilinear electrode bearing structures. Similarly, it should be understood that predicted and targeted lesions may be evaluated and depicted as three dimensional volumes rather than two dimensional areas as appropriate.

Various examples have been described. These and other examples are within the scope of the following claims.

## Claims

1. A system for use in ablating target tissue of a patient, the system comprising:
memory configured to store at least one of anatomical information (210) of a patient or physiological information of the patient, and a numerical model (214); and
processing circuitry (204) communicatively coupled to the memory, the processing circuitry being configured to:
determine, based on the at least one of the anatomical information of the patient or the physiological information of the patient, ablation parameters (218) using the numerical model, the ablation parameters comprising at least one of a suggested positioning of at least one energy delivery element (110) of at least one catheter (102) or an amount of energy to be delivered via the at least one energy delivery element to the target tissue of the patient during ablation; and
output, for display, at least one of a representation of the suggested positioning of the at least one energy delivery element during the ablation, a representation of the target tissue, or a representation of predicted tissue that will be ablated after the delivery of ablation energy via the at least one energy delivery element;
**characterized in that** the processing circuitry is further configured to:
after delivery of ablation energy via the at least one energy delivery element, determine a tissue that was ablated; and
output for display a representation of the tissue that was ablated
and/or
**in that** the processing circuitry is further configured to:
after delivery of ablation energy via the at least one energy delivery element, determine a tissue that was ablated; and
output for display a representation of surrounding tissue, the surrounding tissue comprising tissue adjacent to the tissue that was ablated and not comprising the tissue that was ablated;
wherein the processing circuitry is further configured to:
update the stored anatomical information based on the determination of the tissue that was ablated.

2. The system of claim 1, wherein the numerical model comprises at least one of a machine learning model or an artificial intelligence model.

3. The system of claim 1 or 2, wherein the numerical model is trained on at least one of anatomical information, physiological information, patient information (212), energy delivered during ablation, or tissue that was ablated during ablation for a plurality of patients.

4. The system of one of the claims 1 to 3, further comprising the at least one catheter, wherein the at least one catheter comprises at least one of a pulsed field ablation catheter, a cryoablation catheter, a laser ablation catheter, an ultrasonic ablation catheter, a thermal ablation catheter, a carbon ion beam catheter, or a radio frequency ablation catheter,
and/or
wherein the processing circuitry is further configured to:
control delivery of the at least one catheter into an anatomy of the patient via robotics towards the target tissue.

5. The system of one of the claims 1 to 4, further comprising:
a user interface configured to display the at least one of the representation of the suggested positioning of the at least one energy delivery element during ablation, the representation of the target tissue, or the representation of the predicted tissue that will be ablated after the delivery of the ablation energy via the at least one energy delivery element,
and/or
further comprising:
a user interface (206) configured to receive a user input of a selection of the target tissue, and wherein the processing circuitry is configured to, in response to receiving the user input of the selection of the target tissue, output for display at least one of the representation of the target tissue or the representation of the predicted tissue that will be ablated after the delivery of the ablation energy via the at least one energy delivery element.

6. The system of one of the claims 1 to 5, wherein the processing circuitry is further configured to:
receive the at least one of the anatomical information or the physiological information from one or more of a computed tomography device, a magnetic resonance imaging device, an ultrasound device, a pacing device, an electrophysiology mapping device, or a non-invasive mapping device; and
determine a distance from the at least one energy delivery element to the target tissue, wherein the energy to be delivered to the target tissue during ablation is further based on the distance from the at least one energy delivery element to the target tissue.

7. The system of one of the claims 1 to 6, wherein the processing circuitry is further configured to issue an alert when the at least one energy delivery element is within a treatment range of the target tissue.

8. The system of one of the claims 1 to 7, wherein the processing circuitry is further configured control the at least one energy delivery element to automatically begin delivering energy when the at least one energy delivery element is within the treatment range of the target tissue.

9. The system of one of the claims 1 to 8, wherein the processing circuitry is further configured to control the at least one energy delivery element during delivery of ablation energy to adjust the ablation parameters,
and/or
wherein the processing circuitry is further configured to:
control the at least one energy delivery element to deliver non-therapeutic ablative energy; and
test, based on the delivery of the non-therapeutic energy, assumptions in the numerical model.

10. A non-transitory computer-readable storage medium storing instructions that, when executed in the system of any of the claims 1 to 9, cause the processing circuitry to:
determine, based on at least one of anatomical information of a patient or physiological information of the patient, ablation parameters using a numerical model, the ablation parameters comprising at least one of a suggested positioning of at least one energy delivery element of at least one catheter or an amount of energy to be delivered via the at least one energy delivery element to target tissue of the patient during ablation;
output, for display, a representation of at least one of the suggested positioning of the at least one energy delivery element during the ablation, a representation of the target tissue or a representation of predicted tissue that will be ablated after the delivery of ablation energy via the at least one energy delivery element;
**characterized in that** the instructions further cause the processing circuitry to,
after the delivery of ablation energy via the at least one energy delivery element, determine a tissue that was ablated;
output for display a representation of the tissue that was ablated; and
update the stored anatomical information based on the determination of the tissue that was ablated.

## Patentansprüche

1. System zur Verwendung in der Ablation von Zielgewebe eines Patienten, wobei das System umfasst:
Speicher, der so ausgelegt ist, dass mindestens eines von anatomischen Informationen (210) eines Patienten oder physiologischen Informationen des Patienten sowie ein numerisches Modell (214) gespeichert werden; und
eine Verarbeitungsschaltungsanordnung (204), die kommunikativ mit dem Speicher gekoppelt ist, wobei die Verarbeitungsschaltungsanordnung ausgelegt ist zum:
Bestimmen von Ablationsparametern (218) basierend auf dem mindestens einen von den anatomischen Informationen des Patienten oder den physiologischen Informationen des Patienten unter Verwendung des numerischen Modells, wobei die Ablationsparameter mindestens eines von einer vorgeschlagenen Positionierung von mindestens einem Energieabgabeelement (110) von mindestens einem Katheter (102) oder einer Energiemenge umfassen, die während der Ablation über das mindestens eine Energieabgabeelement an das Zielgewebe des Patienten abgegeben werden soll;
Ausgeben von mindestens einer von einer Darstellung der vorgeschlagenen Positionierung des mindestens eines Energieabgabeelements während der Ablation, einer Darstellung des Zielgewebes oder einer Darstellung des vorhergesagten Gewebes, das nach Abgabe der Ablationsenergie über das mindestens eine Energieabgabeelement ablatiert sein wird, zur Anzeige;
**dadurch gekennzeichnet, dass** die Verarbeitungsschaltungsanordnung weiterhin ausgelegt ist zum:
Bestimmen eines Gewebes, welches ablatiert wurde, nach Abgabe von Ablationsenergie über das mindestens eine Energieabgabeelement; und
Ausgeben einer Darstellung des Gewebes, welches ablatiert wurde, zur Anzeige;
und/oder
dass die Verarbeitungsschaltungsanordnung weiterhin ausgelegt ist zum:
Bestimmen eines Gewebes, welches ablatiert wurde, nach Abgabe von Ablationsenergie über das mindestens eine Energieabgabeelement; und
Ausgeben einer Darstellung des umgebenden Gewebes zur Anzeige, wobei das umgebende Gewebe Gewebe benachbart zu dem Gewebe umfasst, welches ablatiert wurde, und nicht das Gewebe umfasst, welches ablatiert wurde;
wobei die Verarbeitungsschaltungsanordnung weiterhin ausgelegt ist zum:
Aktualisieren der gespeicherten anatomischen Informationen basierend auf der Bestimmung des Gewebes, welches ablatiert wurde.

2. System nach Anspruch 1, wobei das numerische Modell mindestens eines von einem maschinellen Lernmodell oder einem künstlichen Intelligenzmodell umfasst.

3. System nach Anspruch 1 oder 2, wobei das numerische Modell an mindestens einem von anatomischen Informationen, physiologischen Informationen, Patienteninformationen (212), während der Ablation abgegebener Energie, oder Gewebe, das während der Ablation ablatiert wurde, für eine Vielzahl von Patienten trainiert wird.

4. System nach einem der Ansprüche 1 bis 3, weiterhin umfassend den mindestens einen Katheter, wobei der mindestens eine Katheter mindestens einen von einem Pulsfeldablationskatheter, einem Kryoablationskatheter, einem Laser-Ablationskatheter, einem Ultraschall-Ablationskatheter, einem thermischen Ablationskatheter, einem Kohlenstoff-Ionenstrahlkatheter oder einem Hochfrequenz-Ablationskatheter umfasst,
und/oder
wobei die Verarbeitungsschaltungsanordnung weiterhin ausgelegt ist zum:
Steuern von Abgabe des mindestens einen Katheters in eine Anatomie des Patienten hinein mittels Robotik in Richtung Zielgewebe.

5. System nach einem der Ansprüche 1 bis 4, weiterhin umfassend:
eine Benutzeroberfläche, die so ausgelegt ist, dass die mindestens eine von der Darstellung der vorgeschlagenen Positionierung des mindestens einen Energieabgabeelements während der Ablation, der Darstellung des Zielgewebes oder der Darstellung des vorhergesagten Gewebes, welches nach der Abgabe der Ablationsenergie über das mindestens eine Energieabgabeelement ablatiert sein wird, angezeigt wird,
und/oder
weiterhin umfassend:
eine Benutzeroberfläche (206), die ausgelegt ist, um eine Benutzereingabe einer Auswahl des Zielgewebes zu empfangen, und wobei die Verarbeitungsschaltungsanordnung so ausgelegt ist, dass in Reaktion auf Empfangen der Benutzereingabe der Auswahl des Zielgewebes mindestens eine von der Darstellung des Zielgewebes oder der Darstellung des vorhergesagten Gewebes, das nach Abgabe der Ablationsenergie über das mindestens ein Energieabgabeelement ablatiert sein wird, zur Anzeige ausgegeben wird.

6. System nach einem der Ansprüche 1 bis 5, wobei die Verarbeitungsschaltung weiterhin ausgelegt ist zum:
Empfangen der mindestens einen von den anatomischen Informationen oder den physiologischen Informationen von einem oder mehreren von einer Computertomographievorrichtung, einer Magnetresonanztomographievorrichtung, einer Ultraschallvorrichtung, einer Stimulationsvorrichtung, einer Elektrophysiologie-Kartierungsvorrichtung oder einer nicht-invasiven Kartierungsvorrichtung; und
Bestimmen eines Abstands von dem mindestens einen Energieabgabeelement zu dem Zielgewebe, wobei die Energie, die während der Ablation an das Zielgewebe abgegeben werden soll, weiterhin auf dem Abstand von dem mindestens einen Energieabgabeelement zu dem Zielgewebe basiert.

7. System nach einem der Ansprüche 1 bis 6, wobei die Verarbeitungsschaltungsanordnung weiterhin so ausgelegt ist, dass sie einen Alarm ausgibt, wenn sich das mindestens ein Energieabgabeelement innerhalb eines Behandlungsbereichs des Zielgewebes befindet.

8. System nach einem der Ansprüche 1 bis 7, wobei die Verarbeitungsschaltung weiterhin ausgelegt ist, um das mindestens ein Energieabgabeelement zu steuern, um automatisch mit der Energieabgabe zu beginnen, wenn sich das mindestens ein Energieabgabeelement innerhalb des Behandlungsbereichs des Zielgewebes befindet.

9. System nach einem der Ansprüche 1 bis 8, wobei die Verarbeitungsschaltungsanordnung weiterhin so ausgelegt ist, dass sie das mindestens eine Energieabgabeelement während der Abgabe von Ablationsenergie steuert, um die Ablationsparameter anzupassen,
und/oder
wobei die Verarbeitungsschaltungsanordnung weiterhin ausgelegt ist zum:
Steuern des mindestens einen Energieabgabeelements, um nicht-therapeutische Ablationsenergie abzugeben, und
Testen von Annahmen in dem numerischen Modell basierend auf der Abgabe der nicht-therapeutischen Energie.

10. Nicht-flüchtiges computerlesbares Speichermedium, das Anweisungen speichert, die bei Ausführung in dem System nach einem der Ansprüche 1 bis 9 bewirken, dass die Verarbeitungsschaltungsanordnung folgendes ausführt:
Bestimmen von Ablationsparametern basierend auf mindestens einem von anatomischen Informationen eines Patienten oder physiologischen Informationen des Patienten unter Verwendung eines numerischen Modells, wobei die Ablationsparameter mindestens eines von einer vorgeschlagenen Positionierung mindestens eines Energieabgabeelements von mindestens einem Katheter oder einer Energiemenge umfassen, die während der Ablation über das mindestens eine Energieabgabeelement an Zielgewebe des Patienten abgegeben werden soll;
Ausgeben einer Darstellung von mindestens einer von der vorgeschlagenen Positionierungen des mindestens eines Energieabgabeelements während der Ablation, einer Darstellung des Zielgewebes oder einer Darstellung des vorhergesagten Gewebes, das nach der Abgabe von Ablationsenergie über das mindestens ein Energieabgabeelement ablatiert sein wird, zur Anzeige;
**dadurch gekennzeichnet, dass** die Anweisungen weiterhin bewirken, dass die Verarbeitungsschaltung folgendes durchführt:
Bestimmen eines Gewebes, welches ablatiert wurde, nach der Abgabe der Ablationsenergie über das mindestens eine Energieabgabeelement;
Ausgeben einer Darstellung des Gewebes, welches ablatiert wurde, zur Anzeige; und
Aktualisieren der gespeicherten anatomischen Informationen basierend auf der Bestimmung des Gewebes, welches ablatiert wurde.

## Revendications

1. Système destiné à être utilisé dans l'ablation d'un tissu cible d'un patient, le système comprenant :
une mémoire configurée pour stocker des informations anatomiques (210) d'un patient et/ou des informations physiologiques du patient, et un modèle numérique (214) ; et
des circuits de traitement (204) couplés en communication à la mémoire, les circuits de traitement étant configurés pour :
déterminer, sur la base des informations anatomiques du patient et/ou des informations physiologiques du patient, des paramètres d'ablation (218) à l'aide du modèle numérique, les paramètres d'ablation comprenant un positionnement suggéré d'au moins un élément d'application d'énergie (110) d'au moins un cathéter (102) et/ou une quantité d'énergie à appliquer par le biais de l'élément d'application d'énergie au tissu cible du patient pendant l'ablation ; et
sortir, pour affichage, une représentation du positionnement suggéré de l'au moins un élément d'application d'énergie au cours de l'ablation, et/ou une représentation du tissu cible et/ou une représentation du tissu prévu qui sera ablaté après l'application d'énergie d'ablation par le biais de l'au moins un élément d'application d'énergie ;
**caractérisé en ce que** les circuits de traitement sont en outre configurés pour :
après application de l'énergie d'ablation par le biais de l'au moins un élément d'application d'énergie, déterminer un tissu qui a été ablaté ; et
sortir, pour affichage, une représentation du tissu qui a été ablaté
et/ou
**en ce que** les circuits de traitement sont en outre configurés pour :
après application de l'énergie d'ablation par le biais de l'au moins un élément d'application d'énergie, déterminer un tissu qui a été ablaté ; et
sortir, pour affichage, une représentation du tissu environnant, le tissu environnant comprenant un tissu adjacent au tissu qui a été ablaté et ne comprenant pas le tissu qui a été ablaté ;
les circuits de traitement étant en outre configurés pour :
mettre à jour les informations anatomiques stockées en fonction de la détermination du tissu qui a été ablaté.

2. Système selon la revendication 1, dans lequel le modèle numérique comprend un modèle d'apprentissage automatique et/ou un modèle d'intelligence artificielle.

3. Système selon la revendication 1 ou 2, dans lequel le modèle numérique est entraîné sur des informations anatomiques, et/ou des informations physiologiques, et/ou des informations patient (212), et/ou l'énergie appliquée pendant l'ablation, et/ou un tissu qui a été ablaté pendant l'ablation pour une pluralité de patients.

4. Système selon l'une des revendications 1 à 3, comprenant en outre l'au moins un cathéter, dans lequel l'au moins un cathéter comprend un cathéter d'ablation à champ pulsé, et/ou un cathéter de cryoablation, et/ou un cathéter d'ablation laser, un cathéter d'ablation ultrasonique, et/ou un cathéter d'ablation thermique, et/ou un cathéter à faisceau d'ions carbone, et/ou un cathéter d'ablation radiofréquence,
et/ou
les circuits de traitement étant en outre configurés pour :
commander la pose de l'au moins un cathéter dans une partie anatomique du patient par le biais d'un robot vers le tissu cible.

5. Système selon l'une des revendications 1 à 4, comprenant en outre :
une interface utilisateur configurée pour afficher la représentation du positionnement suggéré de l'au moins un élément d'application d'énergie pendant l'ablation, et/ou la représentation du tissu cible et/ou la représentation du tissu prévu qui sera ablaté après l'application de l'énergie d'ablation par le biais de l'au moins un élément d'application d'énergie,
et/ou
comprenant en outre :
une interface utilisateur (206) configurée pour recevoir une entrée utilisateur d'une sélection du tissu cible, et les circuits de traitement étant configurés pour, en réponse à la réception de l'entrée utilisateur de la sélection du tissu cible, sortir, pour affichage, la représentation du tissu cible et/ou la représentation du tissu prévu qui sera ablaté après l'application de l'énergie d'ablation par le biais de l'au moins un élément d'application d'énergie.

6. Système selon l'une des revendications 1 à 5, dans lequel les circuits de traitement sont en outre configurés pour :
recevoir les informations anatomiques et/ou les informations physiologiques provenant d'un dispositif de tomodensitométrie et/ou un dispositif d'imagerie par résonance magnétique et/ou un dispositif à ultrasons et/ou un dispositif de stimulation et/ou un dispositif de cartographie électrophysiologique et/ou un dispositif de cartographie non invasif ; et
déterminer une distance entre l'au moins un élément d'application d'énergie et le tissu cible, l'énergie devant être appliquée au tissu cible pendant l'ablation étant en outre basée sur la distance entre l'au moins un élément d'application d'énergie et le tissu cible.

7. Système selon l'une des revendications 1 à 6, dans lequel les circuits de traitement sont en outre configurés pour émettre une alerte lorsque l'au moins un élément d'application d'énergie se trouve dans une plage de traitement du tissu cible.

8. Système selon l'une des revendications 1 à 7, dans lequel les circuits de traitement sont en outre configurés pour commander l'au moins un élément d'application d'énergie pour qu'il commence automatiquement à appliquer de l'énergie lorsque l'au moins un élément d'application d'énergie se trouve dans la plage de traitement du tissu cible.

9. Système selon l'une des revendications 1 à 8, dans lequel les circuits de traitement sont en outre configurés pour commander l'au moins un élément d'application d'énergie pendant l'application d'énergie d'ablation pour ajuster les paramètres d'ablation,
et/ou
les circuits de traitement étant en outre configurés pour :
commander l'au moins un élément d'application d'énergie pour appliquer une énergie ablative non thérapeutique ; et
tester, sur la base de l'application de l'énergie non thérapeutique, des hypothèses dans le modèle numérique.

10. Support de stockage non transitoire lisible par ordinateur stockant des instructions qui, lorsqu'elles sont exécutées dans le système selon l'une quelconque des revendications 1 à 9, amènent les circuits de traitement à :
déterminer, sur la base d'informations anatomiques d'un patient et/ou d'informations physiologiques du patient, des paramètres d'ablation à l'aide d'un modèle numérique, les paramètres d'ablation comprenant un positionnement suggéré d'au moins un élément d'application d'énergie d'au moins un cathéter et/ou une quantité d'énergie à appliquer par le biais de l'au moins un élément d'application d'énergie au tissu cible du patient pendant l'ablation ;
sortir, pour affichage, une représentation d'au moins un parmi les positionnements suggérés de l'au moins un élément d'application d'énergie au cours de l'ablation, une représentation du tissu cible ou une représentation du tissu prévu qui sera ablaté après l'application d'énergie d'ablation par le biais de l'au moins un élément d'application d'énergie ;
**caractérisé en ce que** les instructions amènent en outre les circuits de traitement à,
après l'application d'énergie d'ablation par le biais de l'au moins un élément d'application d'énergie, déterminer un tissu qui a été ablaté ;
sortir, pour affichage, une représentation du tissu qui a été ablaté ; et
mettre à jour les informations anatomiques stockées en fonction de la détermination du tissu qui a été ablaté.
